# EUROPÄISCHE PATENTSCHRIFT

(11) **EP 0 504 839 B1**
(45) Veröffentlichungstag und Bekanntmachung des Hinweises auf die Patenterteilung: **01.03.1995**
(21) Anmeldenummer: 92104685.0
(22) Anmeldetag: 18.03.1992
(51) Int. Cl.: B01D 65/06, B01D 29/35

(54) **Verfahren zur Abtrennung von Katalysatoren aus Suspensionen durch Filtration**
Separation process of catalysts from suspensions by filtration
Procédé de séparation des catalyseurs de suspensions par filtration

(30) Priorität: 19.03.1991 DE 4108870
(43) Veröffentlichungstag der Anmeldung: 23.09.1992
(73) Patentinhaber: HOECHST AKTIENGESELLSCHAFT, 65926 Frankfurt am Main (DE)
(72) Erfinder: Leupold, Ernst Ingo, Dr., W-6392 Neu-Anspach (DE); Zeisberger, Eduard, Dr., W-6234 Hattersheim am Main (DE); Kauffelt, Manfred, W-6000 Frankfurt am Main (DE); Herzog, Willi, Dr., W-6231 Sulzbach (DE); Dettmeier, Udo, Dr., W-6233 Kelkheim (Taunus) (DE); Weichselbaumer, Georg, Dr., W-6233 Kelkheim (Taunus) (DE)

(56) Entgegenhaltungen:
- EP-A- 0 052 719
- US-A- 3 977 967
- US-A- 4 895 658
- WORLD PATENTS INDEX Week 8005, Derwent Publications Ltd., London, GB; AN 80-08877C

## Beschreibung

Die vorliegende Erfindung betrifft ein Verfahren zur Abtrennung von suspendierten Edelmetallkatalysatoren, die bei der Herstellung von Ethercarbonsäuren durch katalytische Oxidation der entsprechenden Etheralkohole mit Sauerstoff verwendet werden, durch Filtration.

Die katalytische Oxidation von Etheralkoholen gemäß der allgemeinen Gleichung 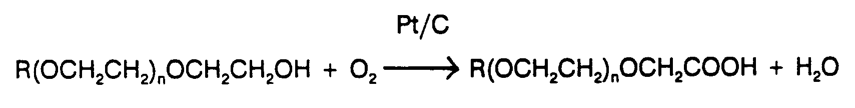
ist seit längerem bekannt und beispielsweise in der DE-C-29 36 123 und der EP-A-206 054 beschrieben. Mit zunehmender Molmasse des Restes R wird jedoch die Abtrennung und damit die vollständige Wiedergewinnung und Rückführung des Edelmetallkatalysators schwieriger.

Es bestand daher die Aufgabe, ein technisch und wirtschaftlich tragbares Verfahren zur Abtrennung von Katalysatoren aus Suspensionen durch Filtration zu entwickeln.

Gegenstand der vorliegenden Erfindung ist nun ein Verfahren zur Abtrennung von Edelmetallkatalysatoren, die bei der Herstellung von Ethercarbonsäuren durch katalytische Oxidation mit suspendiertem Katalysator verwendet werden, das dadurch gekennzeichnet ist, daß man eine Querstromfiltration durchführt und die eingesetzten Membranfilterelemente einer Vor- und gegebenenfalls einer Zwischenbehandlung mit einem unter den Behandlungsbedingungen nicht festen Medium aus einer oder mehreren Carbonsäuren unterwirft.

Bei der Querstromfiltration wird das katalysatorhaltige Reaktionsgemisch mit hoher Überströmgeschwindigkeit tangential zur Membranoberfläche durch das Filterelement gepumpt, wobei das Filtrat quer zur Strömungsrichtung durch die Membranschicht abgeführt wird, wie es im einzelnen in Ullmann's Encyclopedia of Industrial Chemistry, 5th Edition, Band B2, Seiten 10-54 beschrieben wird.

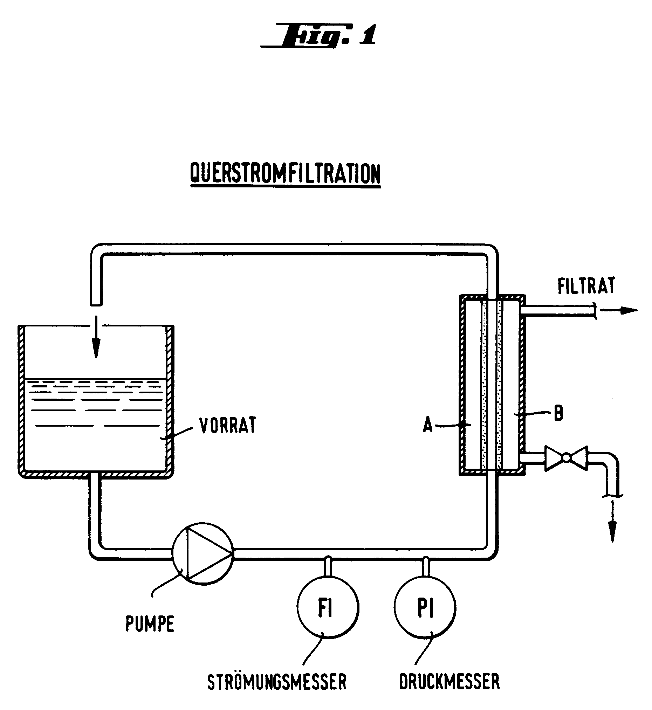

Besonders geeignet sind röhrenförmige Filterelemente mit einer Membranschicht auf der Innenseite der Röhren. Bevorzugte Membran- und Trägermaterialien sind keramische Materialien, α-Al₂O₃ und/oder ZrO₂. Kunststoff- und Kohlenstoffelemente können ebenfalls eingesetzt werden. Membran und Träger können aus verschiedenen Materialien bestehen. Die notwendigen Porengrößen liegen zweckmäßig im Bereich, der für eine Ultrafiltration üblich ist, beispielsweise zwischen etwa 1 und etwa 200 nm. Eine geeignete Appartur ist im Beispiel beschrieben (vgl. hierzu Figur 1).

Bei der erfindungsgemäßen Behandlung des Filterelementes wird dieses zweckmäßig in die flüssige Carbonsäure getaucht. Dies kann z.B. durch direkte Füllung des Filtergehäuses mit der Säure oder nach Ausbau des Filters in einem separaten Gefäß erfolgen. Die Behandlung erfolgt naturgemäß vor dem Filtrationsvorgang und wird zweckmäßig nach längerer Betriebszeit in gewissen Abständen wiederholt. Eine Wiederholung, die beispielsweise nach etwa 30 bis 100 Filtrationen erfolgen kann, ist jedoch nicht zwingend erforderlich; sie kann jedoch bei extremen Filtrationsbedingungen die Leistungsfähigkeit über einen längeren Zeitraum sicherstellen.

Als Carbonsäuren eignen sich insbesondere solche der allgemeinen Formel R-COOH, wobei R einen linearen oder verzweigten Kohlenwasserstoffrest mit 1 bis 18 Kohlenstoffatomen, der mit Hydroxy, Alkoxy, Carboxyl oder Halogen, wie Fluor, Chlor, Brom oder Jod, substituiert sein kann, oder Aryl, Alkyl (C₁-C₉)aryl oder Arylalkyl, z.B. der Benzylrest, wobei das Aryl jeweils 6 bis 16 Kohlenstoffatome enthalten kann, wie Phenyl-, Naphthyl- oder Biphenylreste, und mit den genannten Substituenten substituiert sein kann, bedeutet. Im allgemeinen eignen sich aber alle Carbonsäuren bzw. Gemische oder Lösungen von Carbonsäuren, die bei der Temperatur, bei der die Behandlung des Filterelementes vorgenommen wird, flüssig vorliegen, insbesondere aliphatische und aromatische Verbindungen, wie Ameisensäure, Essigsäure, Propionsäure, Isovaleriansäure, n-Dodecansäure, Benzoesäure und Phenylessigsäure. Es können aber auch ungesättigte, substituierte oder mehrbasische Carbonsäuren, wie Glykolsäure, Methoxyessigsäure, Chloressigsäure, Milchsäure, Crotonsäure, Maleinsäure, eingesetzt werden. Bevorzugt ist wegen der guten Verfügbarkeit und der einfachen Handhabbarkeit Essigsäure.

Die erfindungsgemäße Behandlung erfolgt im allgemeinen bei erhöhter Temperatur, vorzugsweise zwischen etwa 30 und 250°C, besonders bevorzugt zwischen etwa 40 und 160°C. Auch außerhalb dieser Grenzen werden jedoch noch zufriedenstellende Ergebnisse erzielt.

Die Behandlungszeit wird im allgemeinen so gewählt, daß mindestens eine vollständige Permeation des Filters erreicht wird. Im allgemeinen ist eine Behandlungszeit von 0,5 bis 50 h, insbesondere 1 bis 10 h, ausreichend. Das Arbeiten unter erhöhtem Druck, beispielsweise bei 1 bis 10 bar, ist nicht erforderlich, kann aber wegen der schnelleren Permeation des Filters zur Verkürzung der erforderlichen Behandlungsdauer führen. Eine Verlängerung der Behandlungszeit, z.B. während eines Betriebsstillstandes, über mehrere Tage oder Wochen beeinflußt die Wirksamkeit der Filtrierfähigkeit nicht nachteilig. Die Behandlung kann auch mit dampfförmigen Carbonsäuren, beispielsweise durch Überleiten des Dampfes, durchgeführt werden.

Überraschenderweise wird nach dem erfindungsgemäßen Verfahren das Problem der allmählichen Abnahme der Leistung der Filterelemente bei der Filtration, das z.B. bei der Filtration von langkettigen Ethercarbonsäuren, vor allem solcher mit Tensideigenschaften auftritt, durch eine technisch einfache Maßnahme gelöst.

### Beispiel

Eine Lösung, wie sie bei der katalytischen Oxidation von Ethercarbonsäuren mit suspendiertem Katalysator erhalten wird, bestehend aus 25 Gew.-% Ethercarbonsäure der Formel R-(OCH₂CH₂)ₙOCH₂COOH, worin R lineare Alkylgruppen mit einer Verteilung von C₁₂ bis C₁₄ bedeutet und n ein mittlerer Wert von 4 ist, sowie 60 Gew.-% Diethylenglykoldimethylether, 15 Gew.-% Wasser und 5 Gew.-% eines suspendierten handelsüblichen Katalysators (5 Gew.-% Platin auf Aktivkohle) wird 30 Minuten bei 80°C mit Wasserstoff in einer Blasensäule behandelt und anschließend einer Querstromfiltration unterworfen. Das Filterelement wird vor dem Einsatz 8 Stunden bei 70°C in Essigsäure getaucht und anschließend mit Wasser gewaschen. Es besteht aus einem Al₂O₃-Rohr (Durchmesser: 7 mm, Länge: 250 mm), dessen Innenseite aus einer Membranschicht mit Porengrößen von 100 nm (10⁻⁹ m) besteht. Es wird eine Apparatur gemäß Figur 1 verwendet. Die Lösung wird mit einer linearen Strömungsgeschwindigkeit von 5 m/s durch das in einem Gehäuse (A) befindliche Filterelement (B) gepumpt. Es wird ein Druck P₁ von 1,5 bar bei einer Temperatur von 70°C eingestellt. Das Filtrat strömt dann mit einer Geschwindigkeit von 2,0 l/h. Die Aufkonzentrierung des Katalysators gelingt bis zu einem Feststoffanteil von ca. 30 Gew.-%. Dieses Konzentrat wird in die katalytische Oxidation zurückgeführt. Das klare Filtrat gelangt zur weiteren Aufarbeitung. Auch nach 200 Filtrationen mit etwa 2000 l Filtrat wird noch kein Absinken des Filtratstroms beobachtet. Nach jeweils 50 Filtrationen wird eine Zwischenbehandlung (Essigsäure, 2 h, 70°C) durchgeführt.

## Patentansprüche

1. Verfahren zur Abtrennung von Edelmetallkatalysatoren, die bei der Herstellung von Ethercarbonsäuren durch katalytische Oxidation mit suspendiertem Katalysator verwendet werden, dadurch gekennzeichnet, daß man eine Querstromfiltration durchführt und die eingesetzten Membranfilterelemente mit einem unter den Behandlungsbedingungen nicht festen Medium aus einer oder mehreren Carbonsäuren vorbehandelt.

2. Verfahren nach Anspruch 1, dadurch gekennzeichnet, daß die Carbonsäure die allgemeine Formel R-COOH hat, wobei R einen linearen oder verzweigten Kohlenwasserstoffrest mit 1 bis 18 Kohlenstoffatomen, der mit Hydroxy, Alkoxy, Carboxyl oder Halogen substituiert sein kann, oder Aryl, Alkyl(C₁-C₉)aryl oder Arylalkyl, wobei das Aryl jeweils 6 bis 16 Kohlenstoffatome enthalten kann und mit den genannten Substituenten substituiert sein kann, bedeutet.

3. Verfahren nach Anspruch 2, dadurch gekennzeichnet, daß als Carbonsäure Essigsäure eingesetzt wird.

4. Verfahren nach einem oder mehreren der Ansprüche 1 bis 3, dadurch gekennzeichnet, daß man die Membranfilterelemente mit einer oder mehreren Carbonsäuren zwischenbehandelt.

5. Verfahren nach Anspruch 4, dadurch gekennzeichnet, daß man die Zwischenbehandlung nach etwa 30 bis 100 Filtrationen vornimmt.

6. Verfahren nach einem oder mehreren der Ansprüche 1 bis 5, dadurch gekennzeichnet, daß die Membranfilterelemente aus keramischem Material und/oder Kohlenstoff bestehen.

7. Verfahren nach einem oder mehreren der Ansprüche 1 bis 6, dadurch gekennzeichnet, daß die Membranfilterelemente aus ZrO₂ und/oder α-Al₂O₃ bestehen.

8. Verfahren nach einem oder mehreren der Ansprüche 1 bis 7, dadurch gekennzeichnet, daß man die Membranfilterelemente für 0,5 bis 50 h, insbesondere 1 bis 10 h, in die flüssige Carbonsäure taucht.

9. Verfahren nach einem oder mehreren der Ansprüche 1 bis 8, dadurch gekennzeichnet, daß man bei erhöhter Temperatur, bevorzugt zwischen 30 und 250°C, insbesondere zwischen 40 und 160°C, arbeitet.

## Claims

1. A process for separating off rare metal catalysts used in the preparation of ether-carboxylic acids by catalytic oxidation with a suspended catalyst, which comprises carrying out a crossflow filtration and pretreating the membrane filter elements used with a medium which is not solid under the treatment conditions and is composed of one or more carboxylic acids.

2. The process as claimed in claim 1, wherein the carboxylic acid is of the formula R-COOH, where R is a linear or branched hydrocarbon radical having 1 to 18 carbon atoms, which can be substituted by hydroxyl, alkoxy, carboxyl or halogen, or aryl, alkyl(C₁-C₉)aryl or arylalkyl, where the aryl can in each case contain 6 to 16 carbon atoms and can be substituted by the said substituents.

3. The process as claimed in claim 2, wherein the carboxylic acid used is acetic acid.

4. The process as claimed in one or more of claims 1 to 3, wherein the membrane filter elements are subjected to an intermediate treatment with one or more carboxylic acids.

5. The process as claimed in claim 4, wherein the intermediate treatment is carried out after about 30 to 100 filtrations.

6. The process as claimed in one or more of claims 1 to 5, wherein the membrane filter elements are composed of a ceramic material and/or carbon.

7. The process as claimed in one or more of claims 1 to 6, wherein the membrane filter elements are composed of ZrO₂ and/or α-Al₂O₃.

8. The process as claimed in one or more of claims 1 to 7, wherein the membrane filter elements are immersed in the liquid carboxylic acid for 0.5 to 50 hours, in particular 1 to 10 hours.

9. The process as claimed in one or more of claims 1 to 8, which is carried out at an elevated temperature, preferably between 30 and 250°C, especially between 40 and 160°C.

## Revendications

1. Procédé pour la séparation de catalyseurs à base de métaux nobles que l'on utilise dans la préparation d'acides éther-carboxyliques par oxydation catalytique avec un catalyseur en suspension, caractérisé en ce qu'on effectue une filtration tangentielle et on prétraite les élements filtrants membranaires par un milieu non solide dans les conditions du traitement, composé d'un ou de plusieurs acides carboxyliques.

2. Procédé selon la revendication 1, caractérisé en ce que l'acide carboxylique de formule générale R-COOH, où R est un radical hydrocarboné linéaire ou ramifié avec 1 à 18 atomes de carbone, pouvant être substitué par hydroxy, alcoxy, carboxyle ou halogène, ou est un aryle, un (alkyle en C₁-C₉)-aryle ou un arylalkyle, l'aryle pouvant contenir chaque fois de 6 à 16 atomes de carbone ou pouvant être substitué par les substituants cités.

3. Procédé selon la revendication 2, caractérisé en ce qu'on utilise en tant qu'acide carboxylique l'acide acétique.

4. Procédé selon une ou plusieurs des revendications 1 à 3, caractérisé en ce qu'on effectue un traitement intermédiaire des élements filtrants membranaires par un ou plusieurs acides carboxyliques.

5. Procédé selon la revendiaction 4, caractérisé en ce qu'on réalise le traitement intermédiaire après environ 30 à 100 filtrations.

6. Procédé selon une ou plusieurs des revendications 1 à 5, caractérisé en ce que les élements filtrants membranaires se composent de matériau céramique et/ou de carbone.

7. Procédé selon une ou plusieurs des revendications 1 à 6, caractérisé en ce que les éléments filtrants membranaires se composent de ZrO₂ et/ou d'Al₂O₃.

8. Procédé selon une ou plusieurs des revendications 1 à 7, caractérisé en ce qu'on trempe les éléments filtrants membranaires pendant 0,5 à 50 heures, en particulier de 1 à 10 h, dans l'acide carboxylique liquide.

9. Procédé selon une ou plusieurs des revendications 1 à 8, caractérisé en ce qu'on travaille à des températures élevées, de préférence comprises entre 30 et 250°C, plus particulièrement entre 40 et 160°C.
